(19) Europäisches Patentamt
European Patent Office
Office européen des brevets

(11) Veröffentlichungsnummer : **0 457 163 B1**

(12) # EUROPÄISCHE PATENTSCHRIFT

(45) Veröffentlichungstag der Patentschrift :
**06.07.94 Patentblatt 94/27**

(51) Int. Cl.[5] : **C07C 233/47,** C07C 323/59, C07D 207/16, A61K 31/16, A61K 31/395

(21) Anmeldenummer : **91107424.3**

(22) Anmeldetag : **07.05.91**

(54) **Verwendung von Oxalyl-Aminosäurederivaten als Arzneimittel zur Inhibierung der Prolyl-Hydroxylase.**

(30) Priorität : **12.05.90 DE 4015255**

(43) Veröffentlichungstag der Anmeldung :
**21.11.91 Patentblatt 91/47**

(45) Bekanntmachung des Hinweises auf die Patenterteilung :
**06.07.94 Patentblatt 94/27**

(84) Benannte Vertragsstaaten :
**AT BE CH DE DK ES FR GB GR IT LI LU NL SE**

(56) Entgegenhaltungen :
**FR-A- 1 533 817**
**FR-A- 2 010 601**
**CHEMICAL ABSTRACTS, vol. 70, no. 3, 20 Januar 1969 Columbus, Ohio, USASHIRAKAWA KENZO ET AL.: "ALKOXYOXALYLALANINE ESTERS." & JP-A-68010614**

(56) Entgegenhaltungen :
**CHEMICAL ABSTRACTS, vol. 77, no. 19, 06 November 1972 Columbus, Ohio, USA MAEDAITSUTOSHI ET AL.: "SYNTHETIC INTERMEDIATE OF PYRIDOXINE. III. SIMPLE SYNTHESISOF ETHYL N-ETHOXALYLALANINATE AND ETHYL N-FORMYLALANINATE" & BULL. CHEM.SOC. JAP. 1972, 45[6], 1917-18 Seite 342; linke Spalte; ref. no. 125909ZEP 91107424030**
**BIOCHEMISTRY. vol. 27, no. 8, 19 April 1988, EASTON, PA US Seiten 2934 - 2943;CHARLES B. GRISSOM ET AL.: "ISOTOPE EFFECT STUDIES OF THE CHEMICAL MECHANISM OFPIG HEART NADP ISOCITRATE DEHYDROGENASE"**
**FEBS LETTERS. vol. 90, no. 2, Juni 1978, AMSTERDAM NL Seiten 218 - 222;WOLFGANGMÜLLER ET AL.: "REVERSIBLE INHIBITION OF C1Q RELEASE FROM GUINEA PIGMACROPHAGES BY 2,2'-DIPYRIDYL"**

(73) Patentinhaber : **HOECHST AKTIENGESELLSCHAFT**
**D-65926 Frankfurt (DE)**

(72) Erfinder : **Baader, Ekkehard, Dr.**
**Amselweg 14**
**W-6240 Königstein/Taunus (DE)**
Erfinder : **Burghard, Harald, Dr.**
**Feldbergstrasse 96 C**
**W-6384 Schmitten 3 (DE)**
Erfinder : **Günzler-Pukall, Volkmar, Dr.**
**Grosseelheimerstrasse 13**
**W-3550 Marburg (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Oxalyl-aminosäurederivate sind bekannt und beispielsweise beschrieben in FR-A 20 10 601, JP 43/10614 oder Biochemistry, 27 (8), 2934-2943 (1988). Eine Verwendung dieser Verbindungen als Arzneimittel ist im Stand der Technik jedoch nicht beschrieben.

Es wurde nun gefunden, daß Oxalyl-aminosäurederivate der Formel I

$$\begin{array}{ccccccccccc} RO & - & C & - & C & - & N & - & CH & - & C\ -OR' \\ & & \| & & \| & & | & & | & & \| \\ & & O & & O & & R^1 & & R^2 & & O \end{array} \qquad (I)$$

worin

R und R′ gleich oder verschieden sind und $C_1$-$C_6$-Alkyl oder Wasserstoff bedeuten,

$R^1$ Wasserstoff oder $C_1$-$C_4$-Alkyl bedeutet,

$R^2$ Wasserstoff, $C_1$-$C_6$-Alkyl, $C_1$-$C_3$-Alkoxy, Carboxyl, $C_1$-$C_6$-Alkoxycarbonyl, Aryl, SH, $NH_2$ oder Halogen bedeutet, wobei die Alkylreste unsubstituiert oder mit Aryl, OH, SH oder $NH_2$ substituiert sind

oder

$R^1$ und $R^2$ zusammen eine $C_2$-$C_4$-Alkylenkette darstellen

sowie die Verbindungen in ihrer überwiegend reinen D- und L-Form sowie die physiologisch verträglichen Salze ausgezeichnete Inhibitoren der Prolin- und Lysin-Hydroxylase sind.

Die Erfindung betrifft somit die Verwendung der obengenannten Verbindungen als Arzneimittel, insbesondere als Arzneimittel zur Beeinflussung des Stoffwechsels von Kollagen und kollagenähnlichen Stoffen bzw. der Biosynthese von C1q.

Die Erfindung betrifft insbesondere die Verwendung solcher Verbindungen der Formel I, in denen

R und R′ gleich oder verschieden sind und $C_1$-$C_3$-Alkyl, Na oder K bedeuten,

$R^1$ Wasserstoff, Methyl oder Ethyl bedeutet,

$R^2$ Wasserstoff oder $C_1$-$C_4$-Alkyl bedeutet, wobei der Alkylrest unsubstituiert oder mit Phenyl oder SH substituiert ist

oder

$R^1$ und $R^2$ zusammen eine $C_2$- oder $C_3$-Alkylenkette bilden.

Ganz besonders bevorzugt ist die Verwendung von Verbindungen der Formel I, in denen

R und R′ gleich sind und Methyl, Ethyl, Na oder K bedeuten,

$R^1$ Wasserstoff oder Methyl bedeutet,

$R^2$ Wasserstoff, $C_1$-$C_3$-Alkyl, Benzyl oder Thiomethyl bedeutet,

oder

$R^1$ und $R^2$ zusammen eine Ethylenkette bilden.

Alkylketten mit 3 und mehr Kohlenstoffatomen können sowohl geradkettig als auch verzweigt sein. Unter Aryl werden aromatische Kohlenwasserstoffe verstanden, insbesondere Phenyl und Naphthyl. Unter Halogen werden Fluor, Chlor, Brom und Jod verstanden, insbesondere Chlor und Brom.

Die Herstellung von Verbindungen der Formel I ist bekannt und beispielsweise beschrieben in FR-A 20 10 601. Sie gelingt am einfachsten dadurch, daß man 1-3 Äquivalente Aminosäureester-Hydrohalogenid, bevorzugt Hydrochlorid und 1-5 Äquivalente einer Base wie z. B. Carbonat oder Hydrogencarbonat wie Natrium- oder Kaliumcarbonat oder Natrium- oder Kaliumhydrogencarbonat oder tertiäre Amine, wie Triäthylamin, Tributylamin, Äthyldiisopropylamin oder heterocyclische Amine wie N-Alkylmorpholin, Pyridin, Chinolin oder Dialkylaniline zusammengibt.

Gegebenenfalls können auch mehrere Basen gleichzeitig eingesetzt werden. Die Reaktionstemperaturen liegen bei -30°C bis 150°C, bevorzugt bei 20°C bis 100°C. Gegebenenfalls kann auch in einem Lösungsmittel gearbeitet werden, wie Diäthyläther oder Dimethoxyäthan oder Tetrahydrofuran, chlorierten Kohlenwasserstoffen wie Methylenchlorid, Chloroform, Tri- oder Tetrachloräthylen, Benzol, Toluol und auch polaren Lösungsmitteln wie Dimethylformamid, Aceton, Alkoholen wie Methanol oder Ethanol oder Dimethylsulfoxid. Anschließend werden bei Temperaturen zwischen -78°C und 100°C, bevorzugt zwischen -20°C und +20°C langsam 1-3 Äquivalente Oxalsäureesterchlorid zugegeben. Gegebenenfalls kann auch hier mit einem Lösungsmittel wie oben angegeben gearbeitet werden. Die Beendigung der Reaktion läßt sich beispielsweise mittels Dünnschichtchromatographie bestimmen.

Gegebenenfalls kann die Aufarbeitung der Produkte beispielsweise durch Extraktion oder durch Chromatographie z. B. über Kieselgel erfolgen. Das isolierte Produkt kann umkristallisiert werden.

Verbindungen der Formel I mit R und/oder R′ = Alkali wie z. B. Na oder K können z. B. aus den entsprechenden Verbindungen der Formel I mit R und/oder R′ = $C_1$-$C_4$-Alkoxy in alkalischem Medium zu den entsprechenden Salzen verseift werden, z. B. mit NaOH oder KOH in einem niedermolekularen Alkohol wie Methanol oder Äthanol oder in Äthern wie Dimethoxyäthan oder Tetrahydrofuran, gegebenenfalls in Gegenwart von Wasser. In den erhaltenen Salzen läßt sich das Alkalikation nach Ansäuern in Ionenaustauschern in üblicher Weise gegen beliebige Kationen austauschen. Dazu läßt man z. B. die Säuren durch eine mit einem Kationenaustauscher, wie z. B. auf Basis Polystyrol/Divinylbenzol ((R)Amberlite CG-150 oder (R)Dowex-CCR-2) gefüllte Säule laufen. Der Kationenaustauscher ist mit dem gewünschten Kation beladen, z. B. mit Ammoniumionen, die sich von einem primären, sekundären oder tertiären Amin ableiten. Das gewünschte Salz erhält man durch Eindampfen des Eluats.

Ammoniumsalze der Säuren, die sich von einem primären, sekundären oder tertiären Amin ableiten, kann man auch herstellen, indem man die freien Säuren in einer alkoholischen Lösung mit einer äquimolaren Menge des entsprechenden Amins versetzt und das Lösungsmittel eindampft.

Die Herstellung der überwiegend enantiomerenreinen D- oder L-Verbindungen aus den Racematen erfolgt ebenfalls nach literaturbekannten Verfahren, beispielsweise durch fraktionierte Kristallisation oder durch enzymatische Aufarbeitung. Eine andere Möglichkeit besteht in der Direktsynthese der enantiomerenreinen Verbindung aus entsprechenden D- oder L-Vorstufen (Ausgangsverbindungen).

Die erfindungsgemäßen Substanzen sind als reversible Hemmstoffe der Prolylhydroxylase wirksam. Infolgedessen bewirken sie eine selektive Hemmung der kollagenspezifischen Hydroxylierungsreaktion, in deren Verlauf proteingebundenes Prolin durch das Enzym Prolylhydroxylase hydroxyliert wird. Bei Unterbindung dieser Reaktion mittels eines Inhibitors entsteht ein nichtfunktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens. Inhibitoren der Prolylhydroxylase sind deshalb geeignete Werkzeuge in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Außerdem ist bekannt, daß die Inhibierung der Prolylhydroxylase durch bekannte Inhibitoren wie $\alpha,\alpha$-Dipyridyl zu einer Hemmung der C1q-Biosynthese von Makrophagen führt (W. Müller et al., FEBS Lett. 90, 218 f. (1978)). Dadurch kommt es zu einem Ausfall des klassischen Weges der Komplementaktivierung; Inhibitoren der Prolylhydroxylase wirken daher auch als Immunsuppressiva, z. B. bei Immunkomplexkrankheiten.

Die erfindungsgemäßen Substanzen können daher als Fibrosuppressiva, Immunsuppressiva und Antiatherosklerotika eingesetzt werden.

Die antifibrotische Wirkung kann im Modell der Tetrachlorkohlenstoff-induzierten Leberfibrose bestimmt werden. Dazu werden Ratten mit $CCl_4$ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration von 1 - 100 mg/kg wirksam. Ein anderes Modell zur Evaluierung der antibiotischen Wirkung ist das der Bleomycin-induzierten Lungenfibrose wie bei Kelley et al. (J. Lab. Clin. Med. 96, 954, (1980)) beschrieben. Für die Evaluierung der Wirkung der erfindungsgemäßen Verbindungen der Granulationsgewebe kann das Modell des Wattebauschgranuloms, wie bei Meier et al., Experimentia 6, 469 (1950) beschrieben, herangezogen werden. Im folgenden ist die Erfindung anhand von Beispielen näher erläutert.

## BEISPIELE

### Allgemeine Vorschrift zur Herstellung der Verbindungen aus den Beispielen 1 - 6

Ein Äquivalent Aminosäureester-Hydrochlorid, zwei Äquivalente Triethylamin und 2 Äquivalente N,N-Dimethylaminopyridin werden bei Raumtemperatur unter Stickstoff-Atmosphäre in Methylenchlorid vorgelegt. Dann werden bei 0°C - 10°C langsam ein Äquivalent Oxalsäureesterchlorid in Methylenchlorid gelöst zugetropft. Es wird 12 Stunden bei Raumtemperatur gerührt, mit gesättigter Natriumhydrogencarbonat-Lösung versetzt und extrahiert. Die organische Phase wird abgetrennt, mit Natriumchlorid-Lösung gewaschen, mit Magnesiumsulfat getrocknet und eingedampft. Das Rohprodukt wird chromatographiert.

**Beispiel 1**

(N-Oxalyl)-L-alanindimethylester
$R = R' = CH_3$; $R^1 = H$; $R^2 = CH_3$
5 g L-Alaninmethylester-Hydrochlorid und 3,3 ml Oxalsäuremethylesterchlorid ergeben 6 g Beispiel 1 als Öl (Chrom.: EE/$CH_3OH$ 5/1)

**Beispiel 2**

(N-Oxalyl)-L-phenylalanindimethylester
$R = R' = CH_3$; $R^1 = H$;$R^2 = CH_2C_6H_5$
5 g L-Phenylalaninmethylester-Hydrochlorid und 2,2 ml Oxalsäuremethylesterchlorid ergeben 6,5 g Beispiel 2 als Öl (Chrom.: EE/$CH_3OH$ 5/1)

**Beispiel 3**

(N-Oxalyl)-L-glycindimethylester
$R = R' = CH_3$; $R^1 = H$; $R^2 = H$
15 g L-Glycinmethylester-Hydrochlorid und 11 ml Oxalsäuremonomethylesterchlorid ergeben 23 g Beispiel 3; Fp. 49°C; (Chrom.: EE)

**Beispiel 4**

(N-Oxalyl)-L-prolindimethylester
$R = R' = CH_3$; $R^1 = CH_2\text{-}CH_2 = R^2$
2 g L-Prolinmethylester-Hydrochlorid und 2,9 g Oxalsäuremonomethylesterchlorid ergeben 1,5 g als Öl (Chrom.: EE).

**Beispiel 5**

(N-Oxalyl)-L-valindimethylester
$R = R' = CH_3$; $R^1 = H$; $R^2 = -CH(CH_3)_2$
2 g L-Valinmethylester-Hydrochlorid und 2,8 g Oxalsäuremonomethylesterchlorid ergeben 2 g als Öl (Chrom.: CH/EE 1/1).

**Beispiel 6**

(N-Oxalyl)-L-cysteindimethylester
$R = R' = CH_3$; $R^1 = H$; $R^2 = CH_2SH$
2 g L-Cysteinmethylester-Hydrochlorid und 3,9 g Oxalsäuremonomethylesterchlorid ergeben 1,5 g als Öl (Chrom.: CH/EE 1/1).

**Beispiel 7**

(N-Oxalyl)-sarcosindiethylester
$R = R' = C_2H_5$; $R^1 = CH_3$; $R^2 = H$
2 g Sarcosin-ethylester-hydrochlorid in 50 ml Ethanol vorlegen und bei Raumtemperatur eine Lösung aus 3,5 ml (2 Äquivalenten) Oxalsäurediethylester und 1,8 ml Triethylamin in 25 ml Ethanol zutropfen. Für 5 Stunden bei 50°C rühren, dann für 2 Stunden zum Rückfluß erhitzen. Die Lösung wird abgekühlt und zur Trockne eingedampft. Den Rückstand mit Methylenchlorid aufnehmen, einmal mit Wasser waschen, die organische Phase über Magnesiumsulfat trocknen und eindampfen.
Das Rohprodukt wird chromatographiert (EE/CH 1/1)
Ausbeute: 0,35 g

**Allgemeine Vorschrift zur Herstellung der Verbindungen aus Beispiel 8 - 14**

Ein Äquivalent der Verbindung aus den Beispielen 1 - 7 wird bei Raumtemperatur mit 2 Äquivalenten 0,1 N alkoholischer Alkalilauge gelöst. Es wird 12 Stunden bei Raumtemperatur gerührt und zur Trockne eingedampft. Der Rückstand wird zweimal mit Toluol abgeraucht, mehrmals mit Pentan gewaschen und im Hochvakuum getrocknet.

**Beispiel 8**

(N-Oxalyl)-L-alanindikaliumsalz
$R = R' = K$; $R^1 = H$; $R^2 = CH_3$
300 mg der Verbindung aus Beispiel 1 werden mit 32,5 ml 0,1 N ethanolischer Kalilauge umgesetzt.
Ausbeute: 370 mg weiße Kristalle, Fp.: > 300°C

**Beispiel 9**

(N-Oxalyl)-L-phenylalanindinatriumsalz
$R = R^2 = Na$; $R^1 = H$; $R^2 = CH_2C_6H_5$
420 mg der Verbindung aus Beispiel 2 werden mit 32,5 ml 0,1 N methanolischer Natronlauge umgesetzt.
Ausbeute: 440 mg weiße Kristalle, Fp. > 300°C

**Beispiel 10**

(N-Oxalyl)-L-glycindikaliumsalz
$R = R^2 = K$; $R^1 = H$; $R^2 = H$
5,5 g der Verbindung aus Beispiel 3 werden mit 314 ml 0,1 N methanolischer Kalilauge umgesetzt.
Ausbeute: 5,4 g weiße Kristalle, Fp. > 300°C

**Beispiel 11**

(N-Oxalyl)-L-prolindinatriumsalz
$R = R' = Na$; $R^1 = CH_2\text{-}CH_2 = R^2$
300 mg der Verbindung aus Beispiel 4 werden mit 1,5 ml 0,1 N ethanolischer Natronlauge umgesetzt.
Ausbeute: 290 mg weiße Kristalle, Fp. > 300°C

**Beispiel 12**

(N-Oxalyl)-L-valindinatriumsalz
$R = R' = Na$; $R^1 = H$; $R^2 = CH(CH_3)_2$
300 mg der Verbindung aus Beispiel 5 werden mit 14 ml 0,1 N ethanolischer Natronlauge umgesetzt.
Ausbeute: 235 mg weiße Kristalle, Fp.: > 300°C

**Beispiel 13**

(N-Oxalyl)-L-cysteindinatriumsalz
$R = R^1 = Na$; $R^1 = H$; $R^2 = CH_2SH$
300 mg der Verbindung aus Beispiel 6 werden mit 13,7 ml 0,1 N methanolischer Natronlauge umgesetzt
Ausbeute: 300 mg weiße Kristalle, Fp.: > 300°C

**Beispiel 14**

(N-Oxalyl)-sarcosindikaliumsalz
$R = R' = K$; $R^1 = CH_3$; $R^2 = H$
120 mg der Verbindung aus Beispiel 7 werden mit 11,4 ml 0,1 N ethanolischer Kalilauge umgesetzt.
Ausbeute: 130 mg weiße Kristalle, Fp.: > 300°C

Nachfolgend sind die Verbindungen aus den Beispielen 1 - 14 tabellarisch aufgeführt (Tabelle 1)

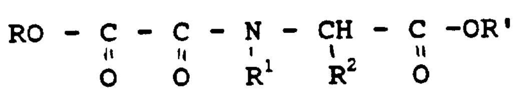

(I)

**TABELLE 1**

| Beispiel | R | R' | $R^1$ | $R^2$ | Fp./Öl |
|---|---|---|---|---|---|
| 1 | $CH_3$ | $CH_3$ | H | $CH_3$ | Öl |
| 2 | $CH_3$ | $CH_3$ | H | $CH_2C_6H_5$ | Öl |
| 3 | $CH_3$ | $CH_3$ | H | H | 49°C |
| 4 | $CH_3$ | $CH_3$ | $CH_2$---- | $CH_2$ | Öl |
| 5 | $CH_3$ | $CH_3$ | H | $CH(CH_3)_2$ | Öl |
| 6 | $CH_3$ | $CH_3$ | H | $CH_2SH$ | Öl |
| 7 | $C_2H_5$ | $C_2H_5$ | $CH_3$ | H | Öl |
| 8 | K | K | H | $CH_3$ | > 300°C |
| 9 | Na | Na | H | $CH_2C_6H_5$ | > 300°C |
| 10 | K | K | H | H | > 300°C |
| 11 | Na | Na | $CH_2$---- | $CH_2$ | > 300°C |
| 12 | Na | Na | H | $CH(CH_3)_2$ | > 300°C |
| 13 | Na | Na | H | $CH_2SH$ | > 300°C |
| 14 | K | K | $CH_3$ | H | > 300°C |

Die Hemmwirkung der erfindungsgemäßen Verbindungen wurde in einem Enzymtest analog der Methode von B. Peterkofsky und R. DiBlasio, Anal. Biochem. 66, 279-286 (1975) bestimmt. Dabei wird unterhydroxyliertes Kollagen mit Prolylhydroxylase in Gegenwart von Eisen(II)ionen, α-Ketoglutarat und Ascorbat enzymatisch hydroxyliert und diejenige Konzentration der zugesetzten erfindungsgemäßen Verbindung bestimmt, die zu einer 80%igen Hemmung der Enzymaktivität führt (der Wert wird als $K_i$ angegeben).

In Tabelle 2 sind die Ergebnisse mit den Verbindungen aus den Beispielen 8 und 10 aufgeführt.

**Tabelle 2 (Salze):**

| Verbindung | $K_i$ [mM] |
|---|---|
| Beispiel 8 | 0,04 |
| Beispiel 10 | 0,01 |

Die Hemmwirkung kann auch in der Zell- oder Gewebekultur bestimmt werden. Dazu können Fibroblasten oder andere Kollagen produzierende Zellen bzw. Calvarien oder andere Kollagen produzierende Organe eingesetzt werden. In Tabelle 3 ist die Hemmwirkung von erfindungsgemäßen Substanzen in der Calvarienkultur zusammengestellt. Angegeben ist die Konzentration, die zu einer 50%igen Absenkung des Hydroxyprolin/Prolin-Quotienten bei metabolischer Markierung mit $^{14}C$-Prolin führt ($IC_{50}$).

**Tabelle 3 (Ester):**

| Verbindung | $IC_{50}$ [mM] |
|---|---|
| Beispiel 1 | 0,35 |
| Beispiel 3 | 0,002 |

## Patentansprüche

### Patentansprüche für folgende Vertragsstaaten : AT, BE, CH, DE, DK, FR, GB, IT, LI, LU, NL, SE

1. Oxalyl-aminosäurederivate der Formel I

$$RO-\underset{\underset{O}{\|}}{C}-\underset{\underset{O}{\|}}{C}-\underset{\underset{R^1}{|}}{N}-\underset{\underset{R^2}{|}}{CH}-\underset{\underset{O}{\|}}{C}-OR' \qquad (I)$$

worin

| | |
|---|---|
| R und R′ | gleich oder verschieden sind und $C_1$-$C_6$-Alkyl oder Wasserstoff bedeuten, |
| $R^1$ | Wasserstoff oder $C_1$-$C_4$-Alkyl bedeutet, |
| $R^2$ | Wasserstoff, $C_1$-$C_6$-Alkyl, $C_1$-$C_3$-Alkoxy, Carboxyl, $C_1$-$C_6$-Alkoxycarbonyl, Aryl, SH, $NH_2$ oder Halogen bedeutet, wobei die Alkylreste unsubstituiert oder mit Aryl, OH, SH oder $NH_2$ substituiert sind |
| oder | |
| $R^1$ und $R^2$ | zusammen eine $C_2$-$C_4$-Alkylenkette darstellen |

sowie die Verbindungen in ihrer überwiegend reinen D- und L-Form sowie die physiologisch verträglichen Salze zur Verwendung als Arzneimittel.

2. Verbindungen der Formel I nach Anspruch 1 zur Verwendung als Arzneimittel, dadurch gekennzeichnet, daß R und R′ gleich oder verschieden sind und $C_1$-$C_3$-Alkyl, Na oder K bedeuten,
   $R^1$ Wasserstoff, Methyl oder Ethyl bedeutet,
   $R^2$ Wasserstoff oder $C_1$-$C_4$-Alkyl bedeutet, wobei der Alkylrest unsubstituiert oder mit Phenyl oder SH substituiert ist
   oder
   $R^1$ und $R^2$ zusammen eine $C_2$- oder $C_3$-Alkylenkette bilden.

3. Verbindungen der Formel I nach Anspruch 1 oder 2 zur Verwendung als Arzneimittel, dadurch gekennzeichnet, daß R und R′ gleich sind und Methyl, Ethyl, Na oder K bedeuten,
   $R^1$ Wasserstoff oder Methyl bedeutet,
   $R^2$ Wasserstoff, $C_1$-$C_3$-Alkyl, Benzyl oder Thiomethyl bedeutet,
   oder
   $R^1$ und $R^2$ zusammen eine Ethylenkette bilden.

4. Verbindungen der Formel I nach einem oder mehreren der Ansprüche 1 bis 3 zur Verwendung als Arzneimittel zur Inhibierung der Enzyme Prolin- oder Lysinhydroxylase.

5. Verbindungen der Formel I nach einem oder mehreren der Ansprüche 1 bis 3 zur Anwendung als Fibrosuppressiva und Immunsuppressiva.

6. Arzneimittel, enthaltend eine Verbindung der Formel I gemäß einem oder mehreren der Ansprüche 1 bis 3 mit verträglichen pharmazeutischen Trägern

7. Verwendung von Verbindungen der Formel I gemäß einem oder mehreren der Ansprüche 1 bis 3 zur Herstellung von fibrosuppressiv und/oder immunsuppressiv wirkenden Arzneimitteln.

8. Verfahren zur Herstellung von Arzneimitteln zur Beeinflussung des Stoffwechsels von Kollagen und kollagenähnlichen Stoffen bzw. der Biosynthese von C1q, dadurch gekennzeichnet, daß man dem Arzneimittel eine Verbindung der Formel I gemäß einem oder mehreren der Ansprüche 1 bis 3 hinzugibt.

### Patentansprüche für folgende Vertragsstaaten : ES, GR

1. Verfahren zur Herstellung von Arzneimitteln zur Beeinflussung des Stoffwechsels von Kollagen und kollagenähnlichen Stoffen bzw. der Biosynthese von Clq, dadurch gekennzeichnet, daß man dem Arzneimittel eine Verbindung der Formel I

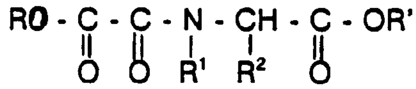

worin

| | |
|---|---|
| R und R′ | gleich oder verschieden sind und $C_1$-$C_6$-Alkyl oder Wasserstoff bedeuten, |
| $R^1$ | Wasserstoff oder $C_1$-$C_4$-Alkyl bedeutet, |
| $R^2$ | Wasserstoff, $C_1$-$C_6$-Alkyl, $C_1$-$C_3$-Alkoxy, Carboxyl, $C_1$-$C_6$-Alkoxycarbonyl, Aryl, SH, $NH_2$ oder Halogen bedeutet, wobei die Alkylreste unsubstituierte oder mit Aryl, OH, SH oder $NH_2$ substituiert sind |

oder

| | |
|---|---|
| $R^1$ und $R^2$ | zusammen eine $C_2$-$C_4$-Alkylenkette darstellen |

sowie die Verbindungen in ihrer überwiegend reinen D- und L-Form sowie die physiologisch verträglichen Salze hinzugibt.

2. Verfahren nach Anspruch 1, dadurch gekennzeichnet, daß R und R′ gleich oder verschieden sind und $C_1$-$C_3$-Alkyl, Na oder K bedeuten,
   $R^1$ Wasserstoff, Methyl oder Ethyl bedeutet,
   $R^2$ Wasserstoff oder $C_1$-$C_4$-Alkyl bedeutet, wobei der Alkylrest unsubstituiert oder mit Phenyl oder SH substituiert ist
   oder
   $R^1$ und $R^2$ zusammen eine $C_2$- oder $C_3$-Alkylenkette bilden.

3. Verfahren nach Anspruch 1 oder 2, dadurch gekennzeichnet, daß R und R′ gleich sind und Methyl, Ethyl, Na oder K bedeuten,
   $R^1$ Wasserstoff oder Methyl bedeutet,
   $R^2$ Wasserstoff, $C_1$-$C_3$-Alkyl, Benzyl oder Thiomethyl bedeutet,
   oder
   $R^1$ und $R^2$ zusammen eine Ethylenkette bilden.

4. Verfahren nach einem oder mehreren der Ansprüche 1 bis 3 zur Herstellung von Arzneimitteln zur Inhibierung der Enzyme Prolin- oder Lysinhydroxylase.

5. Verfahren nach einem oder mehreren der Ansprüche 1 bis 3 zur Anwendung als Fibrosuppressiva und Immunsuppressiva.

6. Verfahren zur Herstellung eines Arzneimittels, enthaltend eine Verbindung der Formel I gemäß einem oder mehreren der Ansprüche 1 bis 5 mit verträglichen pharmazeutischen Trägern.

7. Verwendung von Verbindungen der Formel I gemäß einem oder mehreren der Ansprüche 1 bis 3 zur Herstellung von fibrosuppressiv und /oder immunsuppressiv wirkenden Arzneimitteln.

## Claims

### Claims for the following Contracting States : AT, BE, CH, DE, DK, FR, GB, IT, LI, LU, NL, SE

1. An oxalylamino acid derivative of the formula I

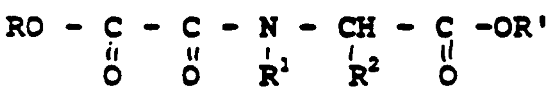

(I)

in which

| | |
|---|---|
| R and R′ | are identical or different and are $C_1$-$C_6$-alkyl or hydrogen, |
| $R^1$ | is hydrogen or $C_1$-$C_4$-alkyl, |
| $R^2$ | is hydrogen, $C_1$-$C_6$-alkyl, $C_1$-$C_3$-alkoxy, carboxyl, $C_1$-$C_6$-alkoxycarbonyl, aryl, SH, $NH_2$ or halogen, where the alkyl radicals are unsubstituted or substituted by aryl, OH, SH or $NH_2$ |

or
$R^1$ and $R^2$ together are a $C_2$-$C_4$-alkylene chain
and the compound in its predominantly pure D- and L-form and the physiologically tolerable salts for use as a pharmaceutical.

2. A compound of the formula I as claimed in claim 1 for use as a pharmaceutical, wherein
R and R′ are identical or different and are $C_1$-$C_3$-alkyl, Na or K,
$R^1$ is hydrogen, methyl or ethyl,
$R^2$ is hydrogen or $C_1$-$C_4$-alkyl, where the alkyl radical is unsubstituted or substituted by phenyl or SH
or
$R^1$ and $R^2$ together form a $C_2$- or $C_3$-alkylene chain.

3. A compound of the formula I as claimed in claim 1 or 2 for use as a pharmaceutical, wherein
R and R′ are identical and are methyl, ethyl, Na or K,
$R^1$ is hydrogen or methyl,
$R^2$ is hydrogen, $C_1$-$C_3$-alkyl, benzyl or thiomethyl,
or
$R^1$ and $R^2$ together form an ethylene chain.

4. A compound of the formula I as claimed in one or more of claims 1 to 3 for use as a pharmaceutical for inhibiting the enzyme proline hydroxylase or lysine hydroxylase.

5. A compound of the formula I as claimed in one or more of claims 1 to 3 for use as a fibrosuppressive and immunosuppressive.

6. A pharmaceutical containing a compound of the formula I as claimed in one or more of claims 1 to 3 with compatible pharmaceutical carriers.

7. The use of compounds of the formula I as claimed in one or more of claims 1 to 3 for the production of pharmaceuticals having fibrosuppressive and/or immunosuppressive activity.

8. A process for the production of pharmaceuticals for influencing the metabolism of collagen and collagen-like substances or the biosynthesis of C1q, which comprises adding a compound of the formula I as claimed in one or more of claims 1 to 3 to the pharmaceutical.

**Claims for the following Contracting States : ES, GR**

1. A process for the production of pharmaceuticals for influencing the metabolism of collagen and collagen-like substances or the biosyntheses of Clq, which comprises adding to the pharmaceutical a compound of the formula I.

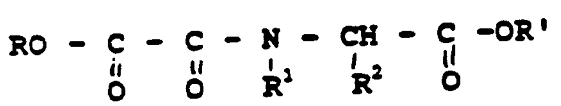

(I)

in which
R and R′ are identical or different and are $C_1$-$C_6$-alkyl or hydrogen,
$R^1$ is hydrogen or $C_1$-$C_4$-alkyl,
$R^2$ is hydrogen, $C_1$-$C_6$-alkyl, $C_1$-$C_3$-alkoxy, carboxyl, $C_1$-$C_6$-alkoxycarbonyl, aryl, SH, $NH_2$ or halogen, where the alkyl radicals are unsubstituted or substituted by aryl, OH, SH or $NH_2$
or
$R^1$ and $R^2$ together are a $C_2$-$C_4$-alkylene chain
and the compounds in their predominantly pure D- and L-form and the physiologically tolerable salts.

2. The process as claimed in claim 1, wherein
R and R′ are identical or different and are $C_1$-$C_3$-alkyl, Na or K,
$R^1$ is hydrogen, methyl or ethyl,

$R^2$ is hydrogen or $C_1$-$C_4$-alkyl, where the alkyl radical is unsubstituted or substituted by phenyl or SH

or

$R^1$ and $R^2$ together form a $C_2$- or $C_3$-alkylene chain.

3. The process as claimed in claim 1 or 2, wherein
   R and R′ are identical and are methyl, ethyl, Na or K,
   $R^1$ is hydrogen or methyl,
   $R^2$ is hydrogen, $C_1$-$C_3$-alkyl, benzyl or thiomethyl,
   or
   $R^1$ and $R^2$ together form an ethylene chain.

4. The process as claimed in one or more of claims 1 to 3 for the production of pharmaceuticals for inhibiting the enzyme proline hydroxylase or lysine hydroxylase.

5. The process as claimed in one or more of claims 1 to 3 for the production of pharmaceuticals for use as fibrosuppressives and immunosuppressives.

6. A process for the production of a pharmaceutical containing a compound of the formula I as in one or more of claims 1 to 3 with compatible pharmaceutical carriers.

7. The use of compounds of the formula I as in one or more of claims 1 to 3 for the production of pharmaceuticals having fibrosuppressive and/or immunosuppressive activity.

## Revendications

### Revendications pour les Etats Contractants suivants : AT, BE, CH, DE, DK, FR, GB, IT, LI, LU, NL, SE

1. Dérivés d'oxalyl-aminoacides de formule I

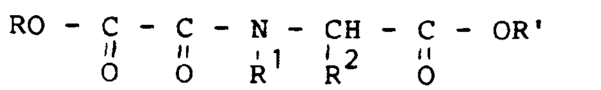

(I)

   dans laquelle
   R et R′ sont identiques ou différents et représentent un groupe alkyle en $C_1$-$C_6$ ou un atome d'hydrogène,
   $R^1$ représente un atome d'hydrogène ou un groupe alkyle en $C_1$-$C_4$,
   $R^2$ représente un atome d'hydrogène ou d'halogène ou un groupe alkyle en $C_1$-$C_6$, alcoxy en $C_1$-$C_3$, carboxy, alcoxy($C_1$-$C_6$)-carbonyle, aryle, SH ou $NH_2$, les groupes alkyle n'étant pas substitués ou étant substitués par un groupe aryle, OH, SH ou $NH_2$, ou
   $R^1$ et $R^2$ représentent ensemble une chaîne alkylène en $C_2$-$C_4$, ainsi que les composés sous leurs formes D et L essentiellement pures, ainsi que les sels physiologiquement acceptables, pour utilisation en tant que médicaments.

2. Composés de formule I selon la revendication 1, pour utilisation en tant que médicaments, caractérisés en ce que
   R et R′ sont identiques ou différents et représentent un groupe alkyle en $C_1$-$C_3$, Na ou K,
   $R^1$ représente un atome d'hydrogène ou le groupe méthyle ou éthyle,
   $R^2$ représente un atome d'hydrogène ou un groupe alkyle en $C_1$-$C_4$, le groupe alkyle n'étant pas substitué ou étant substitué par le groupe phényle ou SH, ou
   $R^1$ et $R^2$ forment ensemble une chaîne alkylène en $C_2$ ou $C_3$.

3. Composés de formule I selon la revendication 1 ou 2, pour utilisaticn en tant que médicaments caractérisés en ce que
   R et R′ sont identiques et représentent le groupe méthyle, éthyle, Na ou K,
   $R^1$ représente un atome d'hydrogène ou le groupe méthyle,

$R^2$ représente un atome d'hydrogène ou un groupe alkyle en $C_1$-$C_3$, benzyle ou thiométhyle, ou

$R^1$ et $R^2$ forment ensemble une chaîne éthylène.

4. Composés de formule I selon une ou plusieurs des revendications 1 à 3, pour utilisation en tant que médicaments destinés à l'inhibition des enzymes proline hydroxylase et lysine hydroxylase.

5. Composés de formule I selon une ou plusieurs des revendications 1 à 3, pour utilisation en tant que fibrosuppresseurs et immunosuppresseurs.

6. Médicament contenant un composé de formule I selon une ou plusieurs des revendications 1 à 3, avec des véhicules pharmaceutiquement acceptables.

7. Utilisation de composés de formule I selon une ou plusieurs des revendications 1 à 3, pour la fabrication de médicaments à action fibrosuppressive et/ou immunosuppressive.

8. Procédé pour la fabrication de médicaments destinés à influencer le métabolisme du collagène et de substances analogues au collagène ou la biosynthèse du C1q, caractérisé en ce qu'on ajoute au médicament un composé de formule I selon une ou plusieurs des revendications 1 à 3.

**Revendications pour les Etats Contractants suivants : ES, GR**

1. Procédé pour la fabrication de médicaments destinés à influencer le métabolisme du collagène et de substances analogues au collagène ou la biosynthèse du C1q, caractérisé en ce que l'on ajoute au médicament un composé de formule I

$$RO-\underset{\underset{O}{\parallel}}{C}-\underset{\underset{O}{\parallel}}{C}-\underset{\underset{R^1}{|}}{N}-\underset{\underset{R^2}{|}}{CH}-\underset{\underset{O}{\parallel}}{C}-OR' \qquad (I)$$

dans laquelle

R et R' sont identiques ou différents et représentent un groupe alkyle en $C_1$-$C_6$ ou un atome d'hydrogène,

$R^1$ représente un atome d'hydrogène ou un groupe alkyle en $C_1$-$C_4$,

$R^2$ représente un atome d'hydrogène ou d'halogène ou un groupe alkyle en $C_1$-$C_6$, alcoxy en $C_1$-$C_3$, carboxy, alcoxy($C_1$-$C_6$)-carbonyle, aryle, SH ou $NH_2$, les groupes alkyle n'étant pas substitués ou étant substitués par un groupe aryle, OH, SH ou $NH_2$, ou

$R^1$ et $R^2$ représentent ensemble une chaîne alkylène en $C_2$-$C_4$, ainsi que les composés sous leurs formes D et L essentiellement pures, ainsi que les sels physiologiquement acceptables.

2. Procédé selon la revendication 1, caractérisé en ce que

R et R' sont identiques ou différents et représentent un groupe alkyle en $C_1$-$C_3$, Na ou K,

$R^1$ représente un atome d'hydrogène ou le groupe méthyle ou éthyle,

$R^2$ représente un atome d'hydrogène ou un groupe alkyle en $C_1$-$C_4$, le groupe alkyle n'étant pas substitué ou étant substitué par le groupe phényle ou SH, ou

$R^1$ et $R^2$ forment ensemble une chaîne alkylène en $C_2$ ou $C_3$.

3. Procédé selon la revendication 1 ou 2, caractérisé en ce que

R et R' sont identiques et représentent le groupe méthyle, éthyle, Na ou K,

$R^1$ représente un atome d'hydrogène ou le groupe méthyle,

$R^2$ représente un atome d'hydrogène ou un groupe alkyle en $C_1$-$C_3$, benzyle ou thiométhyle, ou

$R^1$ et $R^2$ forment ensemble une chaîne éthylène.

4. Procédé selon une ou plusieurs des revendications 1 à 3, pour la fabrication de médicaments destinés à l'inhibition des enzymes proline hydroxylase et lysine hydroxylase.

5. Procédé selon une ou plusieurs des revendications 1 à 3, pour utilisation en tant que fibrosuppresseurs et immunosuppresseurs.

6. Procédé pour la fabrication d'un médicament contenant un composé de formule I selon une ou plusieurs des revendications 1 à 5, avec des véhicules pharmaceutiquement acceptables.

7. Utilisation de composés de formule I selon une ou plusieurs des revendications 1 à 3, pour la fabrication de médicaments à action fibrosuppressive et/ou immunosuppressive.